# EUROPEAN PATENT APPLICATION

(11) **EP 2 028 250 A1**
(43) Date of publication of application: **25.02.2009**
(21) Application number: 07744908.0
(22) Date of filing: 07.06.2007
(51) Int. Cl.: C09K 11/07, C08L 57/06, C12Q 1/42, G01N 21/76, G01N 33/532, C08F 212/14, C08F 212/36, C08F 220/34, C08F 226/06

(54) **LUMINESCENCE ENHANCER**

(30) Priority: 08.06.2006 JP 2006159756
(71) Applicant: FUJIREBIO INC., Chuo-ku, Tokyo 103-0007 (JP)
(72) Inventor: SUGIYAMA, Masami, Chuo-ku, Tokyo 103-0007 (JP)
(74) Representative: HOFFMANN EITLE
(86) International application number: PCT/JP2007/061582
(87) International publication number: WO 2007/142314

(57) **Abstract**

An object of the present invention is to provide a luminescence enhancer by which a luminescence such as chemiluminescence can be enhanced with higher sensitivity and the luminescence can be continued over a prolonged period of time. That is, the present invention provides a luminescence enhancer containing a heteropolymer as an active ingredient, a method for measuring the luminescence using a luminescent substrate and said luminescence enhancer, and a method for analyzing a substance to be measured in a specimen wherein the specimen, an antigen and/or antibody corresponding to a substance to be measured, an antigen and/or antibody labeled-form with an activator and a luminescent substrate are reacted in the presence of said luminescence enhancer to measure the luminescence.

## Description

### TECHNICAL FIELD

The present invention relates to a luminescence enhancer, and preferably relates to a luminescence enhancer by which a luminescence such as chemiluminescence can be enhanced with higher sensitivity and the luminescence can be continued over a prolonged period of time, and use thereof.

### BACKGROUND ART

A method for measuring a chemiluminescence, in which an enzyme is allowed to act upon a chemiluminescent substrate to induce a chemiluminescence has been widely used because the presence or a concentration of a target substance to be measured in a specimen can be measured rapidly with high sensitivity.

Luminescence enhancers for enhancing the luminescence have been actively developed in order to overcome shortcomings, e.g., a short time period of the luminescence in chemiluminescence methods. For example in Patent Document 1 (Patent No. 2660932), a luminescence enhancer used for the method for measuring a peroxidase activity has been disclosed. Also in Patent Document 2 (Patent No. 2984282), organic enhancers utilized for measuring acid phosphatase and alkaline phosphatase have been disclosed.

Patent Document 1: Patent No. 2660932
Patent Document 2: Patent No. 2984282

### DISCLOSURE OF INVENTION

### PROBLEM TO BE SOLVED BY THE INVENTION

However, the above conventional luminescence enhancers have shortcomings; a luminescence enhancing effect is insufficient, and the luminescence is attenuated with time.
The present invention provides a luminescence enhancer by which the aforementioned shortcomings of the conventional art are solved, the luminescence such as chemiluminescence can be enhanced with higher sensitivity and the luminescence can be continued for a prolonged period of time.

### MEANS FOR SOLVING PROBLEM

[1] A luminescence enhancer containing a heteropolymer as an active ingredient.
[2] The luminescence enhancer according to [1], wherein said heteropolymer is an insoluble polymer in water.
[3] The luminescence enhancer according to [1], wherein said heteropolymer is a soluble polymer in water.
[4] The luminescence enhancer according to [1], wherein at least a water-soluble vinyl compound and a cation monomer are included as monomer components which form said heteropolymer.
[5] The luminescence enhancer according to [2], wherein at least a water-soluble vinyl compound, a cation monomer and divinylbenzene are included as monomer components which form said heteropolymer.
[6] The luminescence enhancer according to [4], wherein said water-soluble vinyl compound is a nitrogen-containing heterocyclic vinyl compound.
[7] The luminescence enhancer according to [4], wherein said cation monomer is an ammonium compound and/or phosphonium compound.
[8] The luminescence enhancer according to [4], wherein said cation monomer is a compound represented by the following general formulae (1) and/or (2): 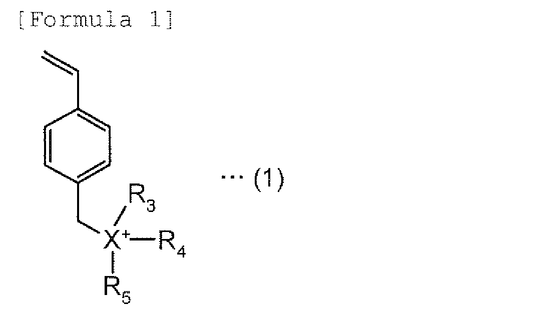 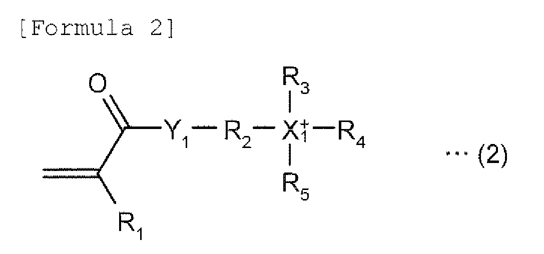 {in the general formula (1) and (2) R₁ represents H or CH₃, each of R₂ to R₅ independently represents H, alkyl having 1 to 16 carbon atoms or N(CH₃)₂ and X₁ represents N or P; and in the general formula (2) Y₁ represents NH, O or CH₂.}
[9] The luminescence enhancer according to [4], wherein said cation monomer is one or more compounds selected from a methacryloyloxytrimethyl ammonium salt, an acrylamide propyltrimethyl ammonium salt, a vinylbenzyltributyl ammonium salt, a vinylbenzyltributyl phosphonium salt, a vinylbenzyltrihexyl phosphonium salt, a vinylbenzyldimethylhexadecyl ammonium salt, a vinylbenzyltrioctyl ammonium salt, a vinylbenzyltrioctyl phosphonium salt and a vinylbenzyltris(dimethylamino) phosphonium salt.
[10] The luminescence enhancer according to [4], wherein said heteropolymer further contains one or more compounds selected from 1-vinyl-2-pyrrolidone, vinylbenzyl bromide, vinylbenzyl chloride, vinylbenzyldimethylamine, vinylbenzyl cyanide, 4-nitro-vinylbenzene, 4-methylstyrene, 3-methylstyrene, 2-methylstyrene, vinyl acetate, 4-aminostyrene, acetic acid 4-vinylphenyl ester, divinyl adipate, benzoic acid vinyl ester, 4-tert-butylbenzoic acid vinyl ester, 1,4-bis [4- (di-p-tolyl-amino) styryl] benzene, chloromethylstyrene, vinyl decanoate, vinyl n-octanoate, vinyl crotonate, vinyl hexanoate, vinyl cinnamate, 2-chlorobenzoic acid vinyl ester, 4-chloromethylstyrene, vinylidene chloride, vinyl methacrylate, 4-methoxystyrene, vinyl laurate, 4-vinylbenzeneboronic acid and vinylpyridine as a monomer component(s).
[11] The luminescence enhancer according to [4], wherein a ratio of an ammonium compound or a ratio of an ammonium compound and a phosphonium compound contained in said heteropolymer is 50 w/v% or less based on the entire heteropolymer.
[12] The luminescence enhancer according to [4], wherein said cation monomers are two or more phosphonium compounds having a different alkyl chain.
[13] A method for measuring a luminescence using a luminescent substrate and the luminescence enhancer according to [4].
[14] The method for measuring a luminescence according to [13], wherein said luminescent substrate is a compound which forms a 1,2-dioxetane structure in a process of a chemiluminescent reaction.
[15] The method for measuring a luminescence according to [13], wherein said luminescent substrate is dioxetanes.
[16] The method for measuring a luminescence according to [15], wherein said dioxetanes are compounds represented by the following general formula (3): 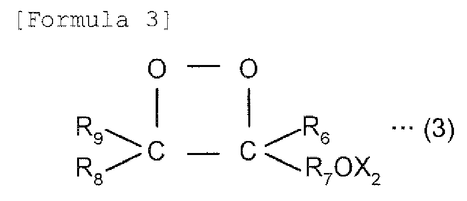 {R₇ represents an aryl group substituted with X₂-oxy, and R₆ is selected from the group consisting of alkyl, alkoxy, aryloxy, dialkylamino, trialkylsilyloxy, arylsilyloxy, aryl and aryl which forms polycyclic aryl substituted with X₂-oxy by binding to aryl R₇, and R₈ and R₉ represent alkyl or heteroalkyl or R₈ and R₉ may be bound each other to form a polycyclic alkylene group.}
[17] The method for measuring a luminescence according to [15], wherein said dioxetanes are compounds represented by the following general formula (4): 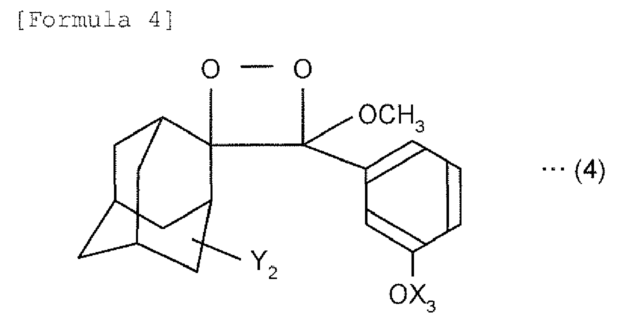 {X₃ represents glycosyl or PO₃Z in which Z represents Na, K or NH₄, and Y₂ represents H, Cl, Br, methyl or methoxy.}
[18] A method for analyzing a substance to be measured in a specimen, wherein the specimen, an antigen and/or antibody corresponding to a substance to be measured, an antigen and/or antibody labeled-form with an activator and a luminescent substrate are reacted in the presence of the luminescence enhancer according to [1] to measure the luminescence.
[19] A kit for analyzing a substance to be measured in a specimen, comprising a solid phase immobilizing an antibody and/or antigen corresponding to a substance to be measured, an antibody and/or antigen labeled-form with an activator and the luminescence enhancer according to [1].

### EFFECT OF THE INVENTION

According to the present invention, it is possible to provide a luminescence enhancer by which the luminescence such as chemiluminescence can be enhanced with higher sensitivity compared with the conventional luminescence enhancers and the luminescence can be continued for a prolonged period of time, and the use thereof.

### BRIEF DESCRIPTION OF DRAWINGS

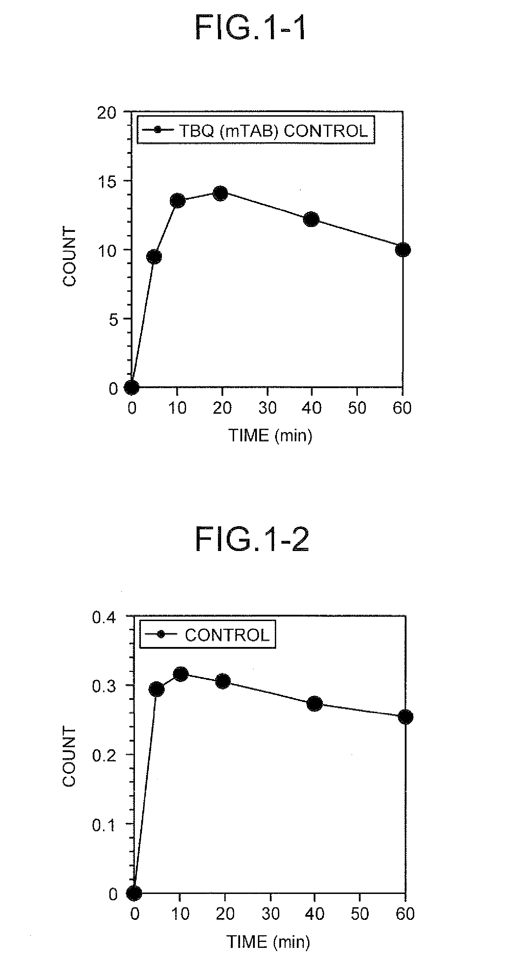

FIG. 1-1 shows the results of measuring luminescence counts in a TBQ substrate solution;
FIG. 1-2 is a graph showing the results of measuring the luminescence counts in a substrate solution containing no enhancer;
FIG. 2 shows the results of measuring the luminescence counts in an enhancer-containing substrate solution prepared in Example 1;
FIG. 3 shows the results of measuring the luminescence counts in an enhancer-containing substrate solution prepared in Example 6;
FIG. 4 shows the results of measuring the luminescence counts in an enhancer-containing substrate solution prepared in Example 7; and
FIG. 5 shows the results of experiments in which an enzymatic reaction was performed using the substrate solution containing no enhancer for 30 to 300 seconds, and then a random copolymer(7:3) enhancer was added to measure the counts.

### EXPLANATIONS OF LETTERS OR NUMERALS

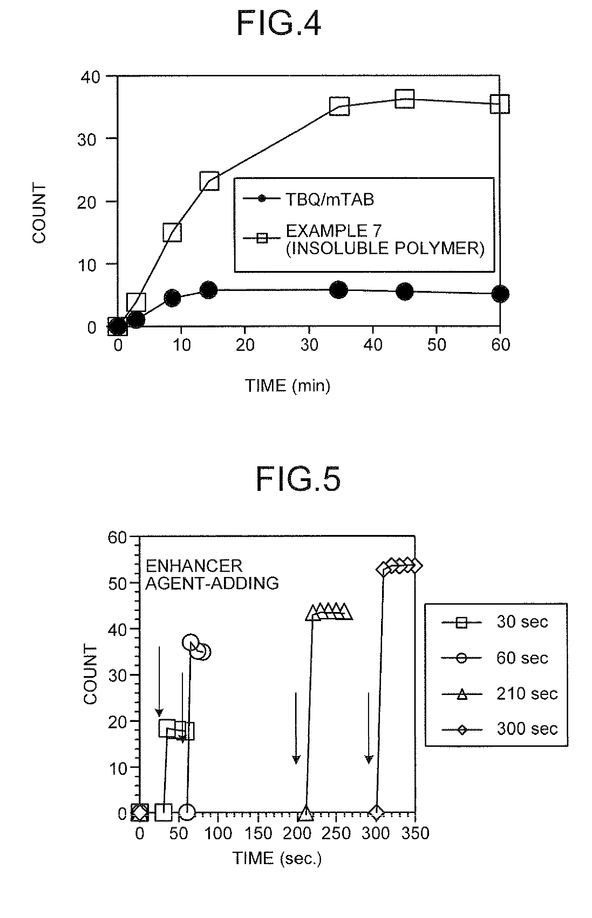

Arrows in FIG. 5 indicate a time when an enhancer was added.

### BEST MODES FOR CARRYING OUT THE INVENTION

The present invention relates to a luminescence enhancer. The luminescence enhancer means a substance which enhances the luminescence, and is also referred to as an enhancer.

The luminescence enhancer of the present invention is characterized by containing a heteropolymer as an active ingredient. The heteropolymer means a polymer composed of two or more monomer components, and can also be reworded as a copolymer. The heteropolymer may be composed of two or more monomer components which align alternately or randomly. In addition, the heteropolymer may be a polymer composed of multiple kinds of components which are heteropolymers themselves (block copolymer), or containing a backbone block having one or more different branch block(s).

The heteropolymer used in the present invention may be soluble in water or insoluble in water. Among the water-insoluble polymers, the polymers referred to as latex polymers in those skilled in the art are preferable. In present invention, being soluble in water refers to dissolving at a concentration of 1% by weight or more in an aqueous solution (e.g., buffer, distilled water) at 4 to 40°C.

A molecular weight and a polymerization degree of the heteropolymer are not particularly limited as long as fulfilling the function as the luminescence enhancer. The molecular weight of the water-soluble heteropolymer is typically 10,000 to 200,000 and preferably 20,000 to 100,000. The polymerization degree is typically 60 to 1,400 and preferably 200 to 700. Meanwhile, for the water-insoluble heteropolymer, particles having diameters of 0.05 to 5.0 µm are preferable while it is difficult to measure its molecular weight and polymerization degree since it is insoluble in most solvents.

A monomer component means a molecule which can become a basic building unit of the polymer. The monomer component is typically the one which forms a polymer by a polymerization reaction. In the case of the present invention, it is necessary to form the heteropolymer, and thus, two or more monomer components having a different structure one another are used. Two or more monomers may be appropriately selected to use so that, for example, each of a water-soluble vinyl compound and a cation monomer are included. By the use of the water-soluble vinyl compound and the cation monomer as one of the monomer components, methyl-3-oxy benzoate, which is a luminescence intermediate to the luminescent substrate and represented by the following structural formula (5), can be drawn to the heteropolymer side.

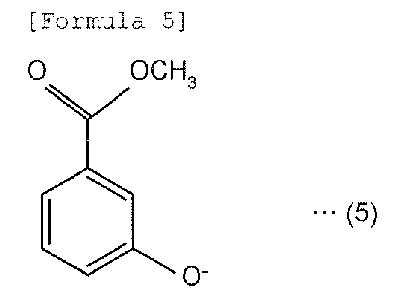

The water-soluble vinyl compounds may include the compounds which are soluble in water and contain vinyl group(s). For example, the compounds such as acrylamide and methacrylamide having an imide group and the compounds such as hydroxyethyl acrylate having a hydroxyl group in addition to nitrogen-containing heterocyclic vinyl compounds may be included. Among them, the nitrogen-containing heterocyclic vinyl compounds are preferable. The nitrogen-containing heterocyclic vinyl compounds may include vinylpyridine, 1-vinyl-2-vinylpyrrolidone, and vinylsuccinimide. Vinylpyridine more specifically may include 4-vinylpyridine, methylvinylpyridine, methoxyvinylpyridine, ethylvinylpyridine and ethoxyvinylpyridine.

The cation monomer which is a compound containing one or more cations, may be any of so-called onium salts and iminium salts, and may be halides such as chloride, bromide and iodide. For example, ammonium compounds, phosphonium compounds, oxonium compounds and sulfonium compounds may be included. Among them, the ammonium compounds and the phosphonium compounds are preferable.

The ammonium compounds may include compounds containing NH₄⁺ (ammonium ion) and compounds where a part or all of H have been substituted with hydrocarbon or OH groups (ammonium derivatives); compounds represented by the general formula R₁₀C=NR₁₀⁺ (iminium); compounds represented by the general formula R-N₂ (diazonium); compounds containing the cation of a non-cyclic nitrogen skeleton, compounds containing the cation of a nitrogen-containing cyclic skeleton; and compounds containing nitrogen-containing resonance stabilized cation (R₁₀ represents a hydrocarbon group such as alkyl and vinyl, when two or more R are bound they may be different one another). Among them, the compounds containing NH₄⁺ (ammonium ion) and the compounds having at least one vinyl group or a substituent containing a vinyl group are preferable. Meanwhile, the phosphonium compounds may include compounds containing PH₄⁺ and compounds containing cation(s) where a part or all of H have been substituted with hydrocarbon or OH groups. Among them, the preferable compounds of the ammonium compounds and the phosphonium compounds can be represented by the above general formulae (1) and (2). In the general formulae (1) and (2), R₁ represents H or CH₃, R₂ to R₅ each independently represent H, alkyl having 1 to 16 carbon atoms or N(CH₃)₂ and X₁ represents N or P, and Y₁ in the general formula (2) represents NH, O or CH₂.
Specific examples of the compounds represented by the general formula (1) may include vinylbenzyltrimethyl ammonium salt, vinylbenzyltripropyl ammonium salt, vinylbenzyltributyl ammonium salt, vinylbenzyldimethylhexadecyl ammonium salt, vinylbenzyltrihexyl ammonium salt, vinylbenzyltrioctyl ammonium salt, vinylbenzyldiethyldecyl ammonium salt, vinylbenzyltrimethyl phosphonium salt, vinylbenzyltripropyl phosphonium salt, vinylbenzyltributyl phosphonium salt, vinylbenzyldimethylhexadecyl phosphonium salt, vinylbenzyltrihexyl phosphonium salt, vinylbenzyltrioctyl phosphonium salt, vinylbenzyldiethyldecyl phosphonium salt and vinylbenzyltris(dimethylamino) phosphonium salt. Specific examples of the compounds represented by the general formula (2) may include acrylamide propyltrimethyl ammonium salt, methacryloyloxytrimethyl ammonium salt, methacrylamide propyltrimethyl ammonium salt, 2-acryloxyethyltrimethyl ammonium salt and diallyldimethyl ammonium salt. Among them, methacryloyloxytrimethyl ammonium salt, acrylamide propyltrimethyl ammonium salt, vinylbenzyltributyl ammonium salt, vinylbenzyltributyl phosphonium salt, vinylbenzyldimethylhexadecyl ammonium salt, vinylbenzyltrioctyl ammonium salt, vinylbenzyltrioctyl phosphonium salt and vinylbenzyltris(dimethylamino) phosphonium salt are preferable. In particular, vinylbenzyltributyl phosphonium salt, acrylamide propyltrimethyl ammonium salt, vinylbenzyltrihexyl phosphonium salt and vinylbenzyltributyl phosphonium salt are preferable.

The oxonium compound may include compounds containing the cation where H₃O⁺ or H has been substituted with an alkyl group. The sulfonium ion may include compounds containing R₁₁S⁺ (R₁₁ represents alkyl or aryl, and three R₁₁ are the same or different).

One of these cation monomers may be used alone, or two or more cation monomers may be used in combination. In particular, it is preferable to combine two or more phosphonium compounds having a different alkyl, chain. "Having a different alkyl chain" refers to having an alkyl chain different in either type or chain length. Preferably, those where the chemical structures other than the alkyl chains are common may be combined to use. Specifically, the combination including the vinylbenzyltrihexyl phosphonium salt and the vinylbenzyltributyl phosphonium salt is preferable, and in particular the combination of only these two is more preferable. Furthermore, it is more preferable to combine and use the vinylbenzyltrihexyl phosphonium salt and the vinylbenzyltributyl phosphonium salt at a ratio of 1 to 5 : 9 to 5, among those, the ratio of 3 to 4 : 7 to 6 is still more preferable.

In the present invention, the other monomers which can form the water-soluble heteropolymer or the water-insoluble polymer, preferably the latex polymer may be used as the monomer component together with the above water-soluble monomer and the cation monomer. As such a monomer component, various compounds such as acrylic-based compounds, styrene-based compounds and vinyl-based compounds may be appropriately selected to use. Specifically, acrylic-based, styrene-based and vinyl-based compounds such as vinylbenzene like divinylbenzene including p-divinylbenzene; vinylbenzyl bromide, vinylbenzyl chloride, vinylbenzyldimethylamine, vinylbenzyl cyanide, 4-nitro-vinylbenzene, 4-methylstyrene, 3-methylstyrene, 2-methylstyrene, vinyl acetate, 4-aminostyrene, acetic acid 4-vinylphenyl ester, divinyl adipate, benzoic acid vinyl ester, 4-tert-butylbenzoic acid vinyl ester, 1,4-bits [4- (di-p-tolyl-amino) styryl] benzene, chloromethylstyrene, vinyl decanoate, vinyl n-octanoate, vinyl crotonate, vinyl n-hexanoate, vinyl cinnamate, 2-chlorobenzoic acid vinyl ester, 4-chloromethylstyrene, vinyl methacrylate, 4-methoxystyrene, vinyl laurate, 4-vinylbenzeneboronic acid, vinylpyridine, 2,2-bis(4-methacryloxyphenyl)propane, N,N'-ethylene bisacrylamide and N,N'-cystaminebisacrylamide may be included. Preferably these compounds may be used in combination of one or more. In particular, in the case of the water-insoluble polymer, it is preferable to include at least divinylbenzene, particularly p-divinylbenzene among the above specific examples. When the phosphonium compound contained in the heteropolymer is the combination of two or more phosphonium compounds having the different alkyl chain, it is not necessary to use the compounds listed above.

A content of the monomer component can be appropriately determined. When the ammonium compound is contained as the cation monomer (containing the ammonium compound and containing no phosphonium compound), a ratio of the ammonium compound which occupies the entire heteropolymer is preferably 50w/v% or less, and particularly preferably 3 to 10w/v%. When the ammonium compound and the phosphonium compound are contained as the cation monomer, a ratio of a sum of the ammonium compound and the phosphonium compound which occupies the entire heteropolymer is preferably 50w/v% or less and particularly preferably 3 to 10w/v%. When the ratio exceeds 50w/v%, it is likely that the effect is reduced. When both the ammonium compound and the phosphonium compound are contained, it is preferable that the sum of them is in the above range. Meanwhile, when the phosphonium compound is contained as the cation monomer (containing the phosphonium compound and containing no ammonium compound), the ratio of the phosphonium compound which occupies the entire heteropolymer may be exceed 50w/v%. However, when two or more phosphonium compounds having the different alkyl chain are used as the phosphonium compounds, the ratio which occupies the entire heteropolymer is preferably 50w/v% or more and more preferably 100w/v%.

The heteropolymer can be produced by polymerizing monomers by standard methods. For example, a polymerization initiator such as 2,2-{azobis(N-carboxyethyl) 2-methylpropionamide is added to and mixed with monomer components, and the polymerization is performed under argon atmosphere at 60 to 100°C for 5 to 24 hours. In the case of the water-insoluble heteropolymer, it is possible to remove the component having a large specific gravity by centrifuging the obtained reaction solution. Meanwhile, in the case of the water-soluble heteropolymer, it is possible to purify by repeating dialysis of the obtained reaction solution.

The luminescence enhancer of the present invention has the aforementioned heteropolymer as an active ingredient, and if necessary, other ingredients may be added thereto. The other luminescence enhancer may also be combined.

The luminescence enhancer of the present invention can enhance the luminescent function of the other substance. Thus, for example, when the luminescence enhancer of the present invention is added to a reaction system in which an enzyme is allowed to act upon a luminescent substrate, luminescence such as chemiluminescence is enhanced. Thus, the luminescence enhancer of the present invention can be used for reliable detection (confirmation, determination) and quantification by an enzyme activity of a target substance to be measured.

That is, the method for measuring the luminescence of the present invention is characterized by using the aforementioned luminescence enhancer upon measuring using a luminescent substrate.

The luminescent substrate may include substances which produce a chemiluminescent reaction by an enzyme, an acid, an alkali, a salt, an enzyme or a catalyst. The luminescent substrate for the chemiluminescent reaction used here may include compounds which form a 1,2-oxetane structure in the process of chemiluminescent reaction, luminol and isoluminol. Among them the compounds which form the 1,2-oxetane structure in the process of chemiluminescent reaction are preferable.
The compounds which form the 1,2-oxetane structure in the process of chemiluminescent reaction may include dioxetanes, Lophine (2,4,5-triphenylimidazole), acridine derivatives (Lucigenin 10,10'-dimethyl-9,9'-biacridinium dinitrate salt, acridinium salts, acridinium ester, etc.), tetraxis(dimethylamino)ethylene, indole (skatole(3-methylindole), etc.), Schiff bases, oxalate derivatives (oxalic acid chloride, oxalate ester, oxalic acid oxamide, etc.) and diphenoyl peroxide. Among them, dioxetanes are the most preferable.

Dioxetanes indicate a generic term of the compounds having a 4-membered peroxide structure.
Preferable specific examples of dioxetanes may include dioxetane derivatives represented by the above general formula (3). In the general formula (3), R₇ represents an aryl group substituted with X₂-oxy group. Such an aryl group may include those which form the 1,2-dioxetane structure which is the unstable intermediate, which then degrade by releasing electron energy to generate light and two carbonyl-containing compounds represented by the following general formula (6), when the reaction is induced by removing X₂ with an activation agent (the activation agent in this specification refers to those having the action to directly or indirectly act upon the luminescent substrate to emit the light) selected from acids, alkalis, salts, enzymes, organic or inorganic catalysts and electron donors. X₂ is a chemically easily reactive group which is removed by an enzyme. R₆ is one selected from the group consisting of aryl groups which form a polycyclic aryl group substituted with X₂-oxy group, and to form said polycyclic aryl group the aryl group has linkages e.g. the spiro linkage to a 1,2-dioxetanes ring to be bound to alkyl, alkoxy, aryloxy, dialkylamino, trialkylsilyloxy, arylsilyloxy group, aryl or the above aryl group R₇. R₈ and R₉ each represent alkyl or heteroalkyl groups, and R₈ and R₉ may be bound each other to form a polycyclic alkylene group which is spiro-bound to the 1,2-dioxetane ring.

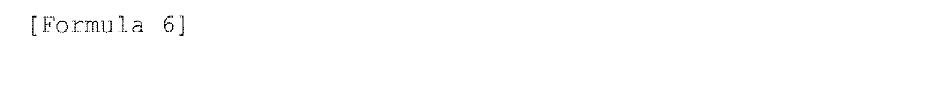

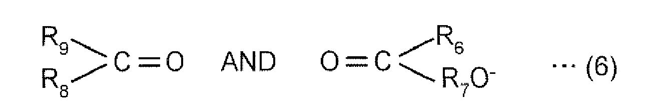

In the general formula (3), when R₆ and R₇ are not bound, this R₆ represents alkyl, alkoxy, aryloxy, dialkylamino, trialkylsilyloxy, arylsilyloxy or aryl group as described above, and preferably represents the lower alkyl or alkoxy group having 1 to 8 carbon atoms. R₆ may also represent aryl, aryloxy or arylsilyloxy having 6 to 20 carbon atoms. When R₆ is bound to R₇ which is aryl group to form a polycyclic aryl group which is spiro-bound to the 1,2-dioxetane ring, it is preferable that the polycyclic aryl group has the carbon atoms up to 30. In this case, the polycyclic aryl group may contain oxygen atoms in place of the carbon atoms like a xanthenyl group, and fluorenyl or xanthenyl where the spiro-bound polycyclic aryl group is spiro-bound at C9 position of the group to the 1,2-dioxetane ring is preferable.

R₇ represents an aryl group substituted with X₂-oxy group (e.g., OX₂ group), and the group containing the aryl group may be phenyl, biphenyl, bound phenyl or other aryl which may contain 6 to 30 carbon atoms and may contain the other substituent. X₂ is the group to be removed from dioxetane by the activation agent in order to degrade the stable dioxetane structure to generate the chemiluminescence (signal). It is preferable that OX₂ group is selected from hydroxy, alkylsilyloxy, arylsilyloxy groups, inorganic oxidic acid salts (particularly, phosphate salt and sulfate salt), pyranoside enzymes, arylcarboxyl ester or alkylcarboxyl ester. When OX₂ group is the hydroxy group, the hydrogen atom in this group is easily reactive with an organic base such as potassium t-butoxide or an inorganic base such as potassium hydroxide, and dioxetane can be degraded by the base to generate the chemiluminescence. When the activation agent is the enzyme typically used well as a label for immunoassays or for detection of DNA probes, OX₂ group having X₂ which is easily reactive with the enzyme could be appropriately selected. For example, when the enzyme is alkaline phosphatase, β-galactosidase, arylesterase or acetylcholine esterase commonly used as a detection tool by a colorimetric substrate or a fluorescent substrate in the immunoassay or the detection of the DNA probe, a phosphate salt, a pyranoside enzyme or an acetate ester group can be selected as the OX₂ group.

R₈ and R₉ each represent alkyl or heteroalkyl group, or may be bound each other to form a cyclic structure or to form a polycyclic alkylene group. The polycyclic alkylene group may contain 6 to 30 carbon atoms, and heteroatoms (nitrogen, oxygen, sulfur or phosphorus). The preferable polycyclic alkylene group is adamantyl. R₈ and R₉ may have substituents as long as the substituent brings the stability to the dioxetane structure and does not impair the stability.

Among the dioxetane derivatives represented by the general formula (3) as above, the suitable luminescent substrate may include the compounds represented by the above general formula (4). In the above general formula (4), X₃ represents glycoside or PO₃Z, and Z represents H, Na, K or NH₄. Y₂ represents H, Cl, Br, methyl or methoxy.

The compounds represented by the general formula (4) may include 3-(4-methoxyspiro[1,2-dioxetane-3,2'-tricyclo[3.3.1^{3.7}]decane]-4-yl)phenylphosphoric acid and disodium salt thereof (AMPPD: registered trade mark of Tropics), 3-(4-methoxyspiro[1,2-dioxetane-3,2'-(5'-chloro)tricycle[3.3.1^{3.7}]decane]-4-yl)phenylphosphoric acid and disodium salt thereof (CSPD: registered trade mark of Tropics), and 3-(4-methoxyspiro[1,2-dioxetane-3,2'-(5'-chloro)tricyclo[3.3,1^{3.7}] decane]-4-yl)phenyl-6-chlorophosphoric acid and disodium salt thereof (CDP-Star: registered trade mark of Tropics). Among them, 3-(4-methoxyspiro[1,2-dioxetane-3,2'-tricyclo[3.3.1^{3.7}]decane]-4-yl)phenylphosphoric acid and a disodium salt thereof are the most preferable. The aforementioned luminescent substrates can be synthesized by chemical reactions, and in addition, commercially available ones may be used. For example, AMPPD supplied from Tropics may be utilized.

Upon performing the method for measuring the luminescence of the present invention, when the enzyme is used, the amount of the aforementioned luminescence enhancer to be used can be typically 2.5 to 150 µM and preferably 5 to 125 µM, and can be 50 to 500 µL and preferably 150 to 250 µL as the ratio to the enzyme. By the use in this range, it is possible to efficiently enhance the luminescence.

The enzyme which can be used for the method for measuring the luminescence of the present invention is not particularly limited as long as the luminescence such as chemiluminescence occurs when the luminescent substrate is allowed to act upon the aforementioned luminescent substrate. For example, alkaline phosphatase, acid phosphatase, galactosidase, glucosidase, glucuronidase or esterase may be included. Preferable examples may include acid phosphatase, alkaline phosphatase, glucosidase, galactosidase and esterase. Among them, alkaline phosphatase is the most preferable. The enzyme can be purified from animals, plants or bacteria by publicly known methods, or the commercially available enzyme may be used. These enzymes may be free or may be bound to the other substance such as proteins e.g., an antibody, an antigen, or a hapten.

In the method for measuring the luminescence of the present invention, conditions are not particularly limited except that the luminescent substrate and the luminescence enhancer are used simultaneously, and the method can be performed in accordance with the ordinary method for measuring the luminescence such as chemiluminescence. That is, the luminescent substrate is first dissolved in water or appropriate buffer, the luminescence enhancer is added to make a luminescent substrate solution, and further the luminescent substrate solution may be mixed with an enzyme solution, or alternatively, the luminescence enhancer and the substrate may be separately added to a specimen solution. A temperature is preferably 20 to 40°C and particularly preferably 30 to 37°C. A time period can be typically 2 to 60 minutes and preferably 5 to 10 minutes.
The luminescence can be measured using a photo counter or a luminometer in the same way as in the detection of the ordinary chemiluminescence. As the luminometer, a commercially available apparatus, e.g., the luminescence measurement apparatus supplied from Aloka Co., Ltd. may be used.

The method for measuring the luminescence of the present invention can be applied to the analysis of various substances including proteins such as antigens and antibodies by utilizing an enzyme as the label (marker), and the present invention also provides the method for analyzing such a protein. That is, the analysis method of the present invention is characterized in that a specimen, an antigen and/or antibody against the substance to be measured, an antigen and/or antibody labeled-form with an activator and a luminescent substrate are reacted in the presence of the aforementioned luminescence enhancer to measure the luminescence. In the method for measuring the luminescence of the present invention, polypeptides, polynucleotides, DNA, RNA and biological trace components in vivo in addition to the proteins can be subject of measuring.

Representative proteins to be subjected to the analysis method of the present invention may include cytokines such as interleukins, various cancer-associated antigens such as cancer embryonic antigen (CEA), antigens such as HBs, various hormones such as thyroid stimulating hormone, antibodies such as IgG or IgM, AFP, insulin and ferritin. The proteins to be measured may be one or, may be two or more.

It need not be considered whether the specimen for the analysis method of the present invention includes the substance to be measured. For example, the specimen can be body fluids such as blood, serum and urine, and samples collected from the body, e.g., cells. The specimen is diluted with water or buffer if necessary, and can also be applied as the specimen solution to the present invention.

The antigen and/or antibody against target substances for measuring is not particularly limited in production methods as long as it exhibits an antigenantibody reaction against a target substance to be detected such as a protein. For example, those obtained by cell culture using standard methods may be utilized, or a recombinant-antibody or an antigen obtained by genetic transformation may be used. The antigen and/or antibody may be a fusion antigen produced by fusing two or more antigens, an antibody fragment such as a Fab fragment and a F(ab)₂ fragment, or a hapten. When the proteins to be measured are two or more, it is necessary to prepare an antigen or antibody against each protein. The antigens and/or antibodies may be bound to a solid phase such as polystyrene beads if necessary.

The antigen and/or antibody labeled-form against a target substance to be measured, which has been labeled with an activator agent, means one that an antibody or an antigen and an enzyme have been bound covalently or non-covalently, and this can be produced by the standard method.
The luminescent substrates are those as described in the previous description of the method for measuring the luminescence of the present invention.

One example of the analysis method of the present invention is described as follows, which indicates a case that the target substance to be measured is a protein and the labeled-form with an activator agent is an enzyme-labeled form.
First, an antibody and/or antigen corresponding to the protein to be subjected to the analysis is reacted with a specimen. When the specimen contains the protein to be subjected, a complex of the protein with the antibody and/or antigen is formed.

Then, an enzyme-labeled form of an antibody and/or antigen corresponding to the antigen and/or antibody to be measured is further reacted with the above complex. By this reaction, a complex of the antigen/antibody-protein-antibody and/or antigen labeled with the enzyme is formed.

Subsequently, a luminescent substrate is further reacted with this complex. By such steps, the sandwich complex of the antigen/antibody-protein-antibody and/or antigen labeled with the enzyme is formed. The aforementioned luminescence enhancer of the present invention is added simultaneously with or rapidly after the addition of the luminescent substrate. Alternatively, the substrate solution containing the luminescence enhancer is added.

Finally, luminescence is detected using a luminometer. When the luminescence is detected, it is evident that the protein is contained in the specimen. Meanwhile, when the luminescence is not detected, it is found that the protein is not contained. The amount of the protein in the specimen can be quantified by previously converting a luminescence amount quantitatively to make a standard curve. This way, according to the analysis method of the present invention, it is possible to detect the presence or absence of the protein in the specimen, determine the type of the protein in the specimen and quantify the protein.

The analysis method of the present invention can be simply performed by a kit comprising a solid phase immobilizing the antibody and/or antigen corresponding to the substance to be measured, the antibody and/or antigen labeled-form with the activator, and the luminescence enhancer of the present invention. The present invention also provides such a kit for the analysis. It is possible to provide the kit for the analysis of the present invention together with a reaction container.

### EXAMPLES

### Example 1: Polymerization of heterocopolymer latex 1

First, in a 1,000 mL recovery flask, 1.74 g of vinylbenzyl cyanide (supplied from Tokyo Chemical industry Co., Ltd.), 4.4 g of 4-vinylpyridine (supplied from Nacalai Tesque Inc), 400 mg of methacryloyloxytrimethyl ammonium (supplied from Wako Pure Chemical Industries Ltd.), 200 µL of p-divinylbenzene (supplied from Sigma Aldrich) and 740 mg of 2,2-{azobis(N-carboxyethyl)2-methylpropionamide} (supplied from Wako Pure Chemical Industries Ltd.) which was a polymerization initiator were added and thoroughly blended; then, 400 mL of water (Milli-Q water) was added therein.

A cooling tube and a three way cock were connected to the recovery flask. A balloon filled with argon gas and a vacuum pump were further connected to the three way cock.
A vacuum using the vacuum pump and a replacement with the argon gas in the connected flask were performed 4 times in 30 minutes by switching the three way cock with immersing the flask in an ultrasonic washer (28 MHz, 20 W).
After 30 minutes, the recovery flask was placed in an oil bath heated at 75°C, and the contents in the flask were reacted for 18 hours to yield a latex. The reaction solution containing the latex was centrifuged at 3,000 rpm to remove a latex component having a large specific gravity.

A obtained supernatant was further centrifuged at 12,000 rpm for 20 minutes. A supernatant was discarded so as to leave a precipitated latex. Ethanol was added to this latex to disperse. After vigorously agitating for 30 minutes, the latex dispersion was centrifuged again at 12,000 rpm for 20 minutes. A supernatant was discarded so as to leave a precipitated latex.
The Milli-Q water was added to the precipitated latex, which was then vigorously agitated until becoming uniform. The latex dispersion was centrifuged again at 12,000 rpm for 20 minutes.
This manipulation was performed 4 times to remove unreacted monomers.
The obtained latex was suspended in Milli-Q water in a minimum amount. Trehalose at a final concentration of 1 w/v% was added to this suspension, which was then lyophilized.
A weight of the obtained latex was 3.3 g by subtracting the added trehalose.

### Example 2: Polymerization of heterocopolymer latex 2

First, in a 1,000 mL recovery flask, 1.74 g of vinylbenzyl cyanide (supplied from Tokyo Chemical industry Co., Ltd.), 4.2 g of 1-vinyl-2-pyrrolidone (supplied from Tokyo Chemical industry Co., Ltd.), 400 mg of methacryloyloxytrimethyl ammonium (supplied from Wako Pure Chemical Industries Ltd.), 200 µL of p-divinylbenzene (supplied from Sigma Aldrich) and 740 mg of 2,2-{azobis(N-carboxyethyl)2-methylpropionamide} (supplied from Wako Pure Chemical Industries Ltd.) which was the polymerization initiator were added and thoroughly blended. 400 mL Of water (Milli-Q water) was added therein.

The cooling tube and the three way cock were connected to the recovery flask. The balloon filled with argon gas and the vacuum pump were further connected to the three way cock.
The vacuum using the vacuum pump and the replacement with the argon gas in the connected flask were performed 4 times in 30 minutes by switching the three way cock with immersing the flask in the ultrasonic washer (28 MHz, 20 W).
After 30 minutes, the recovery flask was placed in the oil bath heated at 75°C, and the contents in the flask were reacted for 18 hours to yield a latex. The reaction solution containing the latex was centrifuged at 3,000 rpm to remove the latex component having the large specific gravity.

A resulting supernatant was further centrifuged at 12,000 rpm for 20 minutes. A supernatant was discarded so as to leave a precipitated latex. Ethanol was added to this latex to disperse. After vigorously agitating for 30 minutes, the latex dispersion was centrifuged again at 12,000 rpm for 20 minutes. A supernatant was discarded so as to leave a precipitated latex.
Milli-Q water was added to the precipitated latex, which was then vigorously agitated until becoming uniform. The latex dispersion was centrifuged again at 12,000 rpm for 20 minutes.
This manipulation was performed 4 times to remove the unreacted monomers.
The resulting latex was suspended in Milli-Q water in the minimum amount. Trehalose at a final concentration of 1 w/v% was added to this suspension, which was then lyophilized.
The weight of the obtained latex was 1.2 g by subtracting the added trehalose.

### Example 3: Polymerization of water-soluble heterocopolymer 3

First, in a 200 mL recovery flask, 1.74 g of vinylbenzyl cyanide (supplied from Tokyo Chemical industry Co., Ltd.), 4.1 g of 2-hydroxyethyl acrylate (supplied from Tokyo Chemical industry Co., Ltd.), 800 mg of methacryloyloxytrimethyl ammonium (supplied from Wako Pure Chemical Industries Lid.), and 74 mg of 2,2-{azobis(N-carboxyethyl)2-methylpropionamide} (supplied from Wako Pure Chemical Industries Ltd.) which was the polymerization initiator were added and thoroughly blended; then, 100 mL of water (Milli-Q water) was added therein.

The cooling tube and the three way cock were connected to the recovery flask. The balloon filled with argon gas and the vacuum pump were further connected to the three way cock.
The vacuum using the vacuum pump and the replacement with the argon gas in the flask were performed 4 times in 10 minutes by switching the three way cock.
After 10 minutes, the recovery flask was placed in the oil bath heated at 75°C, and the contents in the flask were reacted for 4 hours to yield a water-soluble heteropolymer. The obtained water-soluble heteropolymer was put in a dialysis tube with 12,000 cut off, which was dialyzed for three days. In the meantime, an outside solution of the dialysis was changed at least 5 times.

After completing the dialysis, the content was lyophilized.
The weight of the obtained water-soluble heteropolymer was 6.2 g.

### Example 4: Polymerization of water-soluble heterocopolymer 4

First, in a 200 mL recovery flask, 1.74 g of vinylbenzyl cyanide (supplied from Tokyo Chemical industry Co., Ltd.), 4.2 g of 4-vinylpyridine (supplied from Nacalai Tesque Inc), 800 mg of methacryloyloxytrimethyl ammonium (supplied from Wako Pure Chemical Industries Ltd.), and 74 mg of 2,2-{azobis(N-carboxyethyl)2-methylpropionamide} (supplied from Wako Pure Chemical Industries Ltd.) which was the polymerization initiator were added and thoroughly blended; then, 100 mL of water (Milli-Q water) was added therein.

The cooling tube and the three way cock were connected to the recovery flask. The balloon filled with argon gas and the vacuum pump were further connected to the three way cock.
The vacuum using the vacuum pump and the replacement with the argon gas in the connected flask were performed 4 times in 10 minutes by switching the three way cock.
After 10 minutes, the recovery flask was placed in the oil bath heated at 75°C, and the contents in the flask were reacted for 4 hours to yield a water-soluble heteropolymer. The obtained water-soluble heteropolymer was put in the dialysis tube with 12,000 cut off, which was dialyzed for three days. In the meantime, the outside solution of the dialysis was changed at least 5 times.

After completing the dialysis, the content was lyophilized.
The weight of the obtained water-soluble heteropolymer was 5.2 g.

### Example 5: Polymerization of water-soluble heterocopolymer 5

First, in a 200 mL recovery flask, 1.74 g of vinylbenzyl cyanide (supplied from Tokyo Chemical industry Co., Ltd.), 4.4 g of 1-vinyl-2-pyrrolidone (supplied from Tokyo Chemical industry Co., Ltd.), 400 mg of methacryloyloxytrimethyl ammonium (supplied from Wako Pure Chemical Industries Ltd.), and 74 mg of 2,2-{azobis(N-carboxyethyl)2-methylpropionamide} (supplied from Wako Pure Chemical Industries Ltd.) which was the polymerization initiator were added and thoroughly blended; then, 100 mL of water (Milli-Q water) was added therein.

The cooling tube and the three way cock were connected to the recovery flask. The balloon filled with argon gas and the vacuum pump were further connected to the three way cock.
The vacuum using the vacuum pump and the replacement with the argon gas in the connected flask were performed 4 times in 10 minutes by switching the three way cock.
After 10 minutes, the recovery flask was placed in the oil bath heated at 75°C, and the contents in the flask were reacted for 1.5 hours to yield a water-soluble heteropolymer. The obtained water-soluble heteropolymer was put in the dialysis tube with 12,000 cut off, which was dialyzed for three days. In the meantime, the outside solution of the dialysis was changed at least 5 times.

After completing the dialysis, the content was lyophilized.
The weight of the obtained water-soluble heteropolymer was 5.9 g.

### Reference Example 1

AMPPD (supplied from Tropics) which was the luminescent substrate to alkaline phosphatase was dissolved at a concentration of 0.2 mg/mL in 100 mM diethanolamine hydrochloride buffer (pH 10.2). This was made a substrate solution containing no enhancer.
Poly(vinylbenzyltributyl ammonium) (TBQ) at 0.4 mg/mL (supplied from Tropics) together with mTAB (myristyltributyl ammonium Cl) was added to this substrate solution containing no enhancer to make a TBQ substrate solution.

Alkaline phosphatase-labeled anti-interleukin-6 antibody diluted with gelatin-containing Tris hydrochloride buffer to 0.05 µg/mL was used as a specimen.

### Measurement Reference Example 1: Measurement of alkaline phosphatase activity by luminescent substrate

Each 200 µL of the substrate solution containing no enhancer and the TBQ substrate solution prepared in Reference Example 1 was dispensed in a plastic tube, prepared the three tubes for each.
Alkaline phosphatase-labeled anti-interleukin-6 antibody diluted in Reference Example 1 was added at 0.1 µg/mL and 2 µL thereof to each substrate solution to start the reaction. The reaction solution was immediately put in an incubator (dry block) at 37°C to warm up.
In one tube of three tubes of each substrate solution, 2 µL of alkaline phosphatase-labeled anti-interleukin-6 antibody diluted to a concentration at which the measurement was easily performed was added in place of the specimen.
After being warmed up, luminescence counts were measured every an appropriate time using a luminescence measurement apparatus (BLR-20) supplied from Aloka Co., Ltd.
The obtained luminescence counts in the TBQ substrate solution and the luminescence counts in the substrate solution containing no enhancer were shown in FIG. 1-1 and FIG. 1-2, respectively. For the TBQ substrate solution, an enhancement ratio based on the substrate solution containing no enhancer was calculated, and shown in Table 1. That is, the ratio of the signal (luminescence intensity) in the TBQ substrate solution was calculated in the simultaneous measurement, when the signal (control) from the substrate solution containing no enhancer was appointed as 1.

### Measurement Example 1

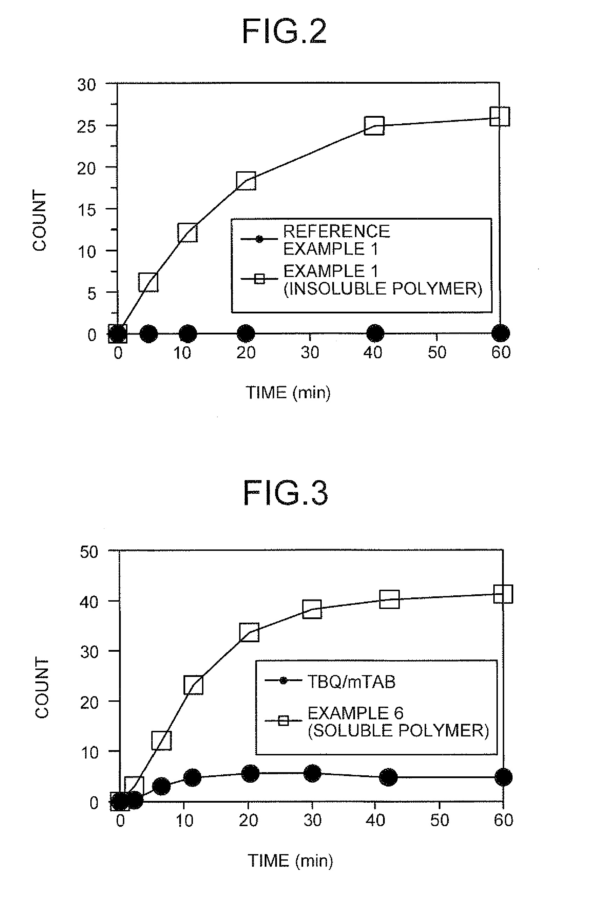

The heterocopolymer latex-1 synthesized in Example 1 was added at 1 w/v% to the substrate solution containing no enhancer prepared in Reference Example 1 to prepare an enhancer-containing substrate solution 1.
The luminescence counts of the alkaline phosphatase activity was measured every an appropriate time by manipulating this substrate solution 1 in the same way as in Reference Example 1. The results have been shown in FIG. 2. The enhancement ratio was calculated in the same way as in Measurement Reference Example 1, and shown in Table 1.

It was revealed from the results of FIG. 2 that the luminescence was widely enhanced and continued for a long time in the case of the substrate solution 1 to which the latex-1 of Example 1 had been added, whereas the luminescence was scarcely detected in the case of the substrate solution containing no enhancer prepared in Reference Example 1. From the comparison with the results of FIG. 1-1, it was revealed that a luminescence enhancement effect of the substrate solution 1 to which the latex-1 of Example 1 had been added was higher than that of the TBQ substrate solution, and was not attenuated with time differently from the TBQ substrate solution.

### Example 6: Polymerization of water-soluble heterocopolymer 6 (phosphonium)

First, to make a phosphonium compound (vinylbenzyltributyl phosphonium), 1.22 g of 4-vinylbenzyl chloride (supplied from Aldrich), 1.6 g of tributylphosphine (supplied from Tokyo Chemical industry Co., Ltd.) and 5 mL of dimethylformamide were put in a 200 mL recovery flask.

The cooling tube and the three way cock were connected to the recovery flask. The balloon filled with argon gas and the vacuum pump were further connected to the three way cock.
The vacuum using the vacuum pump and the replacement with the argon gas in the flask were performed 4 times in 10 minutes by switching the three way cock, and the contents were stirred and reacted at 60°C for 18 hours.

After 18 hours, the cooling tube and the three way cock were removed, and 1.74 g of vinylbenzyl cyanide (supplied from Tokyo Chemical industry Co., Ltd.), 4.4 g of 4-vinylpyridine (supplied from Tokyo Chemical industry Co., Ltd.), and 170 mg of 2,2-{azobis(N-carboxyethyl)2-methylpropionamide }(supplied from Wako Pure Chemical Industries Ltd.) were added; then, 100 mL of water (Milli-Q water) was added therein.
The cooling tube and the three way cock were connected to the recovery flask. The balloon filled with argon gas and the vacuum pump were further connected to the three way cock.
The vacuum using the vacuum pump and the replacement with the argon gas in the flask were performed 4 times in 10 minutes by switching the three way cock.

After 10 minutes, the recovery flask was placed in the oil bath heated at 75°C, and the contents in the flask were reacted for 4 hours to yield a water-soluble heteropolymer. The obtained water-soluble heteropolymer was put in the dialysis tube with 12,000 cut off, which was dialyzed for three days. In the meantime, the outside solution of the dialysis was changed at least 5 times.

After completing the dialysis, the content was lyophilized.
The weight of the obtained water-soluble heteropolymer was 7.2 g.

### Measurement Example 2: Measurement of alkaline phosphatase activity by luminescent substrate

The water-soluble heterocopolymer synthesized in Example 6 was added at 1 w/v% to the substrate solution containing no enhancer prepared in Reference Example 1. Each 200 µL of this solution was dispensed in a plastic tube, prepared the three tubes for each.
Alkaline phosphatase-labeled anti-interleukin-6 antibody diluted in Reference Example 1 was added at 0.05 µg/mL and 2 µL thereof to each substrate solution to start the reaction. The reaction solution was immediately put in the incubator (dry block) at 37°C to warm up.
In one tube of three tubes, 2 µL of alkaline phosphatase-labeled anti-interleukin-6 antibody diluted to the concentration at which the measurement was easily performed was added in place of the specimen.
After being warmed up, the luminescence counts were measured every an appropriate time using the luminescence measurement apparatus (BLR-20) supplied from Aloka Co., Ltd.
The results have been shown in FIG. 3. The enhancement ratio was calculated in the same way as in Measurement Example 1, and shown in Table 1.

As shown in FIG. 3, it was obtained that, the enhancement ratio was 350 times higher in maximum than no addition by the use of the heterocopolymer having phosphonium prepared in Example 6. In addition, it was approximately 6 times the effect of TBQ/mTAB.

### Example 7: Polymerization of water-soluble heterocopolymer 7 (phosphonium)

First, to make a phosphonium compound (vinylbenzyltributyl phosphonium), 1.22 g of 4-vinylbenzyl chloride (supplied from Aldrich), 1.6 g of tributylphosphine (supplied from Tokyo Chemical industry Co., Ltd.) and 5 mL of dimethylformamide were put in a 200 mL recovery flask.

The cooling tube and the three way cock were connected to the recovery flask. The balloon filled with argon gas and the vacuum pump were further connected to the three way cock.
The vacuum using the vacuum pump and the replacement with the argon gas in the flask were performed 4 times in 10 minutes by switching the three way cock, and the contents were stirred and reacted at 60°C for 18 hours.

After 18 hours, the cooling tube and the three way cock were removed, and 1.74 g of vinylbenzyl cyanide (supplied from Tokyo Chemical industry Co., Ltd.), 4.4 g of 4-vinylpyridine (supplied from Tokyo Chemical industry Co., Ltd.), 200 µL of p-divinylbenzene (supplied from Sigma Aldrich) and 170 mg of 2,2-{azobis(N-carboxyethyl)2-methylpropionamide} (supplied from Wako Pure Chemical Industries Ltd.) were added; then, 400 mL of 1 w/v% methylated β-cyclodextrin (supplied from Ensuiko Sugar Refining Co., Ltd.) in Milli-Q water was added therein.
The cooling tube and the three way cock were connected to the recovery flask. The balloon filled with argon gas and the vacuum pump were further connected to the three way cock.
The vacuum using the vacuum pump and the replacement with the argon gas in the connected flask were performed 4 times in 20 minutes by switching the three way cock with immersing the flask in the ultrasonic washer (28 MHz, 20 W).

After 20 minutes, the recovery flask was placed in the oil bath heated at 75°C, and the contents in the flask were reacted for 4 hours to yield a water-soluble heteropolymer. The obtained water-soluble heteropolymer was put in the dialysis tube with 12,000 cut off, which was dialyzed for three days. In the meantime, the outside solution of the dialysis was changed at least 5 times.

After completing the dialysis, the content was lyophilized.
The weight of the obtained water-soluble heteropolymer was 4.8 g.

Measurement Example 3: Measurement of alkaline phosphatase activity by luminescent substrate
The heterocopolymer latex 7 synthesized in Example 7 was added at 0.4 w/v% to the substrate solution containing no enhancer prepared in Reference Example 1. Each 200 µL of this solution was dispensed in a plastic tubes, prepared the three tubes for each.
Alkaline phosphatase-labeled anti-interleukin-6 antibody diluted in Reference Example 1 was added at 0.05 µg/mL and 2 µL thereof to each substrate solution to start the reaction. The reaction solution was immediately put in the incubator (dry block) at 37°C to warm up.
In one tube of three tubes, 2 µL of alkaline phosphatase-labeled anti-interleukin-6 antibody diluted to the concentration at which the measurement was easily performed was added in place of the specimen.
After being warmed up, the luminescence counts were measured every an appropriate time using the luminescence measurement apparatus (BLR-20) supplied from Aloka Co., Ltd.
The results have been shown in FIG. 4. The enhancement ratio was calculated in the same way as in Measurement Example 1, and shown in Table 1.

As shown in FIG. 4, it was obtained that, the enhancement ratio was 200 times higher in maximum than no addition by the use of the heterocopolymer having phosphonium prepared in Example 7. In addition, it was approximately 6 times the effect of TBQ/mTAB.

**TABLE 1**

| | ENHANCEMENT RATIO |
|---|---|
| NO ADDING | 1 |
| TBQ/mTAB | 34 |
| MEASURING FOR EXAMPLE-1 | 469 |
| MEASURING FOR EXAMPLE-2 | 280 |
| MEASURING FOR EXAMPLE-3 | 220 |

### Example 8

### (1) Synthesis of vinylbenzyltrihexyl phosphonium

In a 50 mL recovery flask, 1.5 g of 4-vinylbenzyl cyanide (supplied from Tokyo Chemical industry Co., Ltd.) and 2.9 g of trihexylphosphine (supplied from Tokyo Chemical industry Co., Ltd.) were put. The three way cock was attached to this flask, and the balloon filled with argon gas was connected to one of the cock. Deaeration and the replacement with the argon gas were performed three times, and the contents were stirred at room temperature for 5 days.

After 5 days, the reaction mixture was concentrated using an evaporator, and the concentration was stopped when a viscosity became high. To this flask, 100 mL of diethyl ether (supplied from Wako Pure Chemical Industries Ltd.) was added with stirring. Scaled crystal was precipitated, and this was collected by filtrating with G-4 glass filter. The crystal was thoroughly washed with diethyl ether.
After washing, the crystal was dried in a desiccator. An objective product in a dry weight of about 1.8 g was obtained.

### (2) Synthesis of vinylbenzyltributyl phosphonium

In a 50 mL recovery flask, 1.5 g of 4-vinylbenzyl cyanide (supplied from Tokyo Chemical industry Co., Ltd.) and 2.0 g of tributylphosphine (supplied from Tokyo Chemical industry Co., Ltd.) were put. The three way cock was attached to this flask, and the balloon filled with the argon gas was connected to one of the cock. Deaeration and the replacement with the argon gas were performed three times, and the contents were stirred at room temperature for 2 days.

After 2 days, the reaction mixture was concentrated using the evaporator, and the concentration was stopped when the viscosity became high. To this flask, 20 mL of Milli-Q water was added with stirring. This aqueous solution was put in a 100 mL separatory funnel, and 50 mL of ethyl acetate (supplied from Wako Pure Chemical Industries Ltd.) was further added to extract unreacted compounds. This manipulation was performed three times.

After completing the washing with ethyl acetate, the aqueous solution was concentrated using the evaporator. The concentration was stopped when the crystal was precipitated.
The crystal was dried in the desiccator to yield an objective product in the dry weight of about 2.3 g.

### (3) Production of random copolymer of vinylbenzyltrihexyl phosphonium and vinylbenzyltributyl phosphonium (7:3)

In a 100 mL recovery flask, 1.8 g of vinylbenzyltrihexyl phosphonium produced in (1), 640 mg of vinylbenzyltributyl phosphonium produced in (2) and 85 mg of 2,2-azobis(N-carboxyethyl)2-methylpropionamide (supplied from Wako Pure Chemical Industries Ltd.) were put. Further, 20 mL of ethanol (supplied from Wako Pure Chemical Industries Ltd.) and 20 mL of Milli-Q water were added therein. The cooling tube connected to the three way cock was attached to this recovery flask. A rubber balloon filled with 1 L of the argon gas was attached to the three way cock.

Deaeration and the replacement with the argon gas were performed three times, and the contents were reacted at 75°C for 24 hours with stirring. After completing the reaction, a reaction product was concentrated to about 1/2 using the evaporator, and then dialyzed in the dialysis tube with 12,000 cut off for 3 days with sometimes changing the outside solution.

### Measurement Example 4

AMPPD (supplied from Tropics) was dissolved at 0.2 mg/mL in 0.1 M triethanolamine hydrochloride buffer pH 10.5 (1 mM-Mg) to make a substrate solution. The random copolymerization enhancer made in Example 8 was dissolved at 0.55 w/v% in this substrate solution to make an enhancer substrate solution. A substrate solution containing no enhancer was also prepared.

To 200 µL of this substrate solution containing the enhancer or the substrate solution containing no enhancer, 2 µL of alkaline phosphatase-labeled anti-interleukin-6 antibody (supplied from Fujirebio, Inc.) at 0.05 µg/mL was added. Immediately after the addition, the luminescence was measured every 20 seconds by the luminescence measurement apparatus (BLR-20) supplied from Aloka Co., Ltd. The results have been shown in FIG. 2. The enhancement ratio was obtained by calculating the ratio of the count when the enhancer concentration was 0.55 W/v% based on the count which was made 1 when no enhancer was added. The alkaline phosphatase activity was measured using the substrate solution containing the random copolymer (7:3) enhancer and the substrate solution containing no enhancer, and the results have been compared in the Table.

As the last-in method, the random copolymer enhancer made in Example 8 was added at 0.55 w/v% of final concentration 30, 60, 210 or 300 seconds after the start of the enzymatic reaction, and the count for one second was measured every 20 seconds 5 times. The results have been shown in FIG. 5.

**TABLE 2**

| | ENHANCER CONCENTRATION (0.55% (w/v)) |
|---|---|
| ENZYME-REACTING TIME (min) | ENHANCEMENT RATIO |
| 0 | 11 |
| 1 | 230 |
| 4 | 304 |
| 6 | 318 |
| 9 | 326 |
| 14 | 350 |
| 17 | 352 |

## Claims

1. A luminescence enhancer comprising a heteropolymer as an active ingredient.

2. The luminescence enhancer according to claim 1,
wherein said heteropolymer is an insoluble polymer in water.

3. The luminescence enhancer according to claim 1,
wherein said heteropolymer is a soluble polymer in water.

4. The luminescence enhancer according to claim 1,
wherein at least a water-soluble vinyl compound and a cation monomer are included as monomer components which form said heteropolymer.

5. The luminescence enhancer according to claim 2,
wherein at least a water-soluble vinyl compound, a cation monomer and divinylbenzene are included as monomer components which form said heteropolymer.

6. The luminescence enhancer according to claim 4,
wherein said water-soluble vinyl compound is a nitrogen-containing heterocyclic vinyl compound.

7. The luminescence enhancer according to claim 4,
wherein said cation monomer is an ammonium compound and/or phosphonium compound.

8. The luminescence enhancer according to claim 4,
wherein said cation monomer is a compound represented by the following general formulae (1) and/or (2): 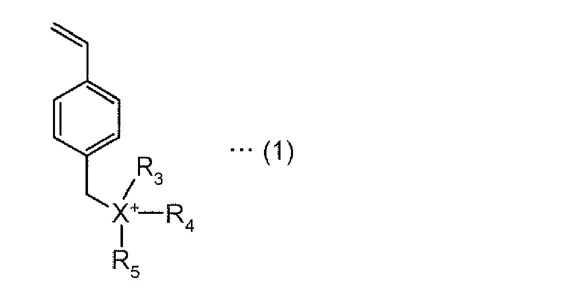 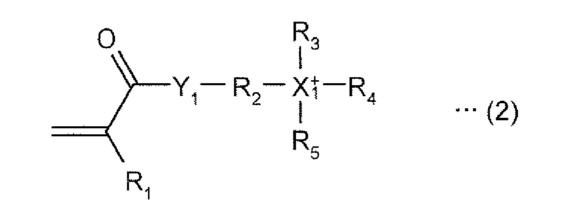 [in the formula (1) and (2), R₁ represents H or CH₃, each of R₂ to R₅ independently represents H, alkyl having 1 to 16 carbon atoms or N(CH₃)₂ and X₁ represents N or P; and in the general formula (2), Y₁ represents NH, O or CH₂.]

9. The luminescence enhancer according to claim 4,
wherein said cation monomer is one or more compounds selected from a methacryloyloxytrimethyl ammonium salt, an acrylamide propyltrimethyl ammonium salt, a vinylbenzyltributyl ammonium salt, a vinylbenzyltributyl phosphonium salt, a vinylbenzyltrihexyl phosphonium salt, a vinylbenzyldimethylhexadecyl ammonium salt, a vinylbenzyltrioctyl ammonium salt, a vinylbenzyltrioctyl phosphonium salt and a vinylbenzyltris(dimethylamino) phosphonium salt.

10. The luminescence enhancer according to claim 4,
wherein said heteropolymer further includes one or more compounds selected from 1-vinyl-2-pyrrolidone, vinylbenzyl bromide, vinylbenzyl chloride, vinylbenzyldimethylamine, vinylbenzyl cyanide, 4-nitro-vinylbenzene, 4-methylstyrene, 3-methylstyrene, 2-methylstyrene, vinyl acetate, 4-aminostyrene, acetic acid 4-vinylphenyl ester, divinyl adipate, benzoic acid vinyl ester, 4-tert-butylbenzoic acid vinyl ester, 1,4-bits [4- (di-p-tolyl-amino) styryl] benzene, chloromethylstyrene, vinyl decanoate, vinyl n-octanoate, vinyl crotonate, vinyl hexanoate, vinyl cinnamate, 2-chlorobenzoic acid vinyl ester, 4-chloromethylstyrene, vinylidene chloride, vinyl methacrylate, 4-methoxystyrene, vinyl laurate, 4-vinylbenzeneboronic acid and vinylpyridine as a monomer component(s).

11. The luminescence enhancer according to claim 4,
wherein a ratio of an ammonium compound or a ratio of an ammonium compound and a phosphonium compound contained in said heteropolymer is 50 w/v% or less based on the entire heteropolymer.

12. The luminescence enhancer according to claim 4,
wherein said cation monomers are two or more phosphonium compounds having a different alkyl chain.

13. A method for measuring a luminescence using a luminescent substrate and the luminescence enhancer according to claim 1.

14. The method for measuring a luminescence according to claim 13, wherein said luminescent substrate is a compound which forms a 1,2-dioxetane structure in a process of a chemiluminescent reaction.

15. The method for measuring a luminescence according to claim 13, wherein said luminescent substrate is dioxetanes.

16. The method for measuring a luminescence according to claim 15, wherein said dioxetanes are compounds represented by the following general formula (3): 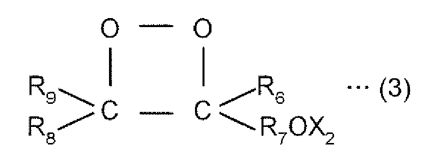 [in the formula(3), R₇ represents an aryl group substituted with X₂-oxy, and R₆ is selected from the group consisting of alkyl, alkoxy, aryloxy, dialkylamino, trialkylsilyloxy, arylsilyloxy, aryl and aryl which forms polycyclic aryl substituted with X₂-oxy by binding to aryl R₇, and R₈ and R₉ represent alkyl or heteroalkyl or R₈ and R₉ may be bound each other to form a polycyclic alkylene group.]

17. The method for measuring a luminescence according to claim 15, wherein said dioxetanes are compounds represented by the following general formula (4): 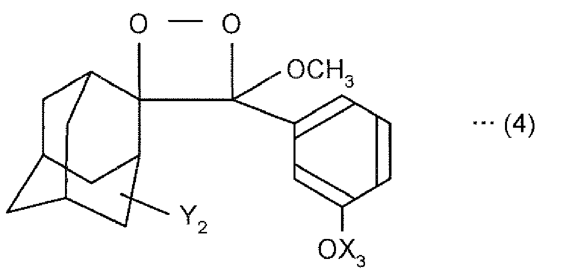 [in the formula(4), X₃ represents glycosyl or PO₃Z in which Z represents Na, K or NH₄, and Y₂ represents H, Cl, Br, methyl or methoxy.]

18. A method for analyzing a substance to be measured in a specimen, wherein the specimen, an antigen and/or antibody corresponding to a substance to be measured, an antigen and/or antibody labeled-form with an activator and a luminescent substrate are reacted in the presence of the luminescence enhancer according to claim 1 to measure the luminescence.

19. A kit for analyzing a substance to be measured in a specimen, comprising a solid phase immobilizing an antibody and/or antigen corresponding to a substance to be measured, an antibody and/or antigen labeled-form with an activator and the luminescence enhancer according to claim 1.
